**Europäisches Patentamt**

**European Patent Office**

**Office européen des brevets**

(19)

(11) Veröffentlichungsnummer : **0 025 835**
**B1**

# (12) EUROPÄISCHE PATENTSCHRIFT

(45) Veröffentlichungstag der Patentschrift :
**23.05.84**

(51) Int. Cl.³ : **A 61 F   1/03**

(21) Anmeldenummer : **80104321.7**

(22) Anmeldetag : **23.07.80**

(54) **Oberschenkelteil einer Hüftgelenkendoprothese.**

(30) Priorität : **03.09.79 DE 2935511**

(43) Veröffentlichungstag der Anmeldung :
**01.04.81 Patentblatt 81/13**

(45) Bekanntmachung des Hinweises auf die Patenterteilung : **23.05.84 Patentblatt 84/21**

(84) Benannte Vertragsstaaten :
**AT CH DE FR GB IT LI SE**

(56) Entgegenhaltungen :
**CH-A-   552 383**
**DE-A- 2 138 146**
**DE-A- 2 645 100**
**DE-A- 2 805 868**
**DE-A- 2 838 335**
**DE-U- 7 436 033**
**FR-A- 2 361 093**

(73) Patentinhaber : **Schunk & Ebe GmbH**
**Rodheimer Strasse 59-61**
**D-6301 Heuchelheim (DE)**

(72) Erfinder : **Hüttinger, K. J., Prof. Dr.**
**Allensteiner Strasse 17**
**D-7500 Karlsruhe (DE)**
Erfinder : **Brückmann, H., Dr.**
**Professorenweg 29**
**D-6300 Giessen (DE)**

EP 0 025 835 B1

**Beschreibung**

Die Erfindung betrifft ein Oberschenkelteil einer Hüftgelenkendoprothese, bestehend aus einem faserverstärkten, gekrümmten Endoprothesenschaft mit unidirektionaler oder teilweise unidirektionaler Anordnung der Fasern in Längsrichtung des Schaftes, der ein oberes Schaftteil, ein stark gekrümmtes Mittelteil und ein sich zum freien Ende hin verjüngendes unteres Schaftteil umfaßt, und einem aus Kugel und Kragenteil bestehenden Endoprothesenkopf, wobei der mit dem Endoprothesenkopf fest verbundene Endoprothesenschaft keinen Kragen aufweist und wobei die durch die Senkrechte durch den Kugelmittelpunkt auf die Kopfbasis gegebene Kopfachse und die durch die Hauptorientierung der Fasern im oberen Schaftteil und den Mittelpunkt der durch die Kopfbasisfläche bestimmten virtuellen Schnittfläche durch den Schaft gegebene obere Schaftachse nicht identisch sind, wobei die Kopfachse und die obere Schaftachse einen spitzen Winkel einschließen.

Die heute verwendeten Hüftkopfendoprothesen haben Oberschenkelteile, die nahezu ausschließlich aus Metall bestehen. Als metallische Werkstoffe werden gegossene oder geschmiedete Sonderlegierungen, wie z. B. Cobalt-Chrom-Molybdän- oder Titan-Aluminium-Vanadium-Legierungen verwendet. Die metallischen Oberschenkelteile werden entweder aus einem Stück gefertigt, oder der aus einer Gußlegierung bestehende Kopf wird auf den aus einer Schmiedelegierung bestehenden Schaft aufgeschweißt.

Eine Variation des rein metallischen Oberschenkelteils besteht darin, daß an Stelle des Metallkopfes ein Kopf aus hochdichter Aluminiumoxidkeramik verwendet wird (DE-A-21 34 316). Für die Verbindung beider Teile wurden Klemm- bzw. Schraubverbindungen vorgeschlagen (CH-A-552 383 ; DE-U-74 36 033 ; DE-B-25 48 591). Aus der DE-U-74 36 033 ist bekannt, das obere Schaftteil konisch auszubilden.

Da bei der rein metallischen und auch bei der Metall-Aluminiumoxidkeramik-Endoprothese sowohl Schaft als auch Kopf aus im wesentlichen isotropen Werkstoffen bestehen, ist auf eine besondere Orientierung der Werkstoffe in Bezug auf die Prothesengestaltung nicht zu achten.

In neuster Zeit wurden als Materialien für Endoprothesenschäfte faserverstärkte Werkstoffe vorgeschlagen, bei denen es sich in erster Linie um kohlenstofffaserverstärkte Kohlenstoffe handelt. Diese Werkstoffe weisen neben der hervorragenden Biokompatibilität herausragende statische und Dauerwechsel-Festigkeiten auf. Zur Realisierung einer hohen Schaftfestigkeit ist jedoch Voraussetzung, daß die Faserverstärkung vorwiegend unidirektional erfolgt. Bei nicht ausschließlich unidirektionaler Verstärkung muß auf jeden Fall gewährleistet sein, daß die über alle Fasern gemittelte Abweichung von der Hauptorientierung gering bleibt.

Zur Kombination mit einem Schaft aus kohlenstofffaserverstärktem Kohlenstoff wurde als Material für den Endoprothesenkopf ein polykristalliner Kohlenstoff bzw. ein Siliziumcarbid-Kohlenstoff-Verbundwerkstoff vorgeschlagen (s. die am 13.3.80 veröffentlichte, keinen Stand der Technik bildende DE-A-28 38 333). Als vorteilhafte Verbindung von Schaft und Kopf wurde nach der EP-A-0 009 148, die unter Art 54 (3) EPÜ fällt, des weiteren eine Siliziumcarbidverbindungsschicht vorgeschlagen, die die formschlüssige Berührungsfläche der Prothesenteile kraftschlüssig verbindet. Aus dieser EP-A-0 009 148 geht ein Oberschenkelteil einer Hüftgelenkendoprothese hervor, das aus einem faserverstärkten, gekrümmten Endoprothesenschaft mit unidirektionaler oder teilweise unidirektionaler Anordnung der Fasern in Längsrichtung des Schaftes, der ein oberes, im wesentlichen konisches Schaftteil, ein stark gekrümmtes Mittelteil und ein sich zum freien Ende hin verjüngendes unteres Schaftteil umfaßt, und einem Endoprothesenkopf aus polykristallinem, isotropem, keramischem Material besteht, wobei der Endoprothesenkopf Kugel und Kragenteil umfaßt und der in den Kopf bis in die Kugel hineinragende und mit diesem fest verbundene Endoprothesenschaft keinen Kragen aufweist.

Aus der DE-A-2 805 868 ist ein Oberschenkelteil einer Hüftgelenkendoprothese mit den im Oberbegriff des Anspruchs 1 genannten Merkmalen bekannt, das also aus einem faserverstärkten, gekrümmten Endoprothesenschaft mit unidirektionaler oder teilweise unidirektionaler Anordnung der Fasern in Längsrichtung des Schaftes, der ein oberes Schaftteil, ein stark gekrümmtes Mittelteil und ein sich zum freien Ende hin verjüngendes unteres Schaftteil umfaßt, und aus einem aus Kugel und Kragenteil bestehenden **Endoprothesenkopf besteht, wobei der mit dem Endoprothesenkopf fest verbundene Endoprothesenschaft keinen Kragen aufweist.** Dieser bekannte Oberschenkelteil der Hüftgelenkendoprothese erfüllt die Bedingung, daß die durch die Senkrechte durch den Kugelmittelpunkt auf die Kopfbasis gegebene Kopfachse und die durch die Hauptorientierung der Fasern im oberen Schaftteil und den Mittelpunkt der durch die Kopfbasisfläche bestimmten virtuellen Schnittfläche durch den Schaft gegebene obere Schaftachse nicht identisch sind. Die Kopfachse und die obere Schaftachse können einen spitzen Winkel einschließen (DE-A-2 805 868). Gemäß der CH-A-552 383 kann die obere Schaftachse gegenüber der Kopfachse parallel zur konkaven Seite des Schaftes verschoben sein.

Bei der Herstellung von Prothesen durch Kombination von faserverstärkten Werkstoffen mit vorwiegend unidirektionaler Anordnung der Fasern und polykristallinen, keramischen und vorwiegend isotropen Werkstoffen hat sich gezeigt, daß bekannte Prothesenkonstruktionen nicht verwendet werden können. Dies hängt einmal damit zusammen, daß unidirektional oder vorwiegend unidirektional mit Fasern verstärkte Werkstoffe zwar eine außerordentliche Festigkeit in Faserrichtung, dafür jedoch eine um nahezu eine Größenordnung geringere Festigkeit senkrecht zur Faserorientierung besitzen. Der zweite Grund ergibt sich hieraus, daß polykristalline, keramische Materialien zum Teil zwar außerordentlich hohe Druckkräfte, jedoch nur sehr geringe Zugkräfte aufzunehmen vermögen. Schließlich ist zu berücksichtigen, daß polykristalline, keramische Werkstoffe außerordentlich kerbempfindlich sind.

Aufgabe der vorliegenden Erfindung ist es, ein Oberschenkelteil einer Hüftgelenkendoprothese der eingangs genannten Art zu schaffen, das den speziellen mechanischen Eigenschaften des vorwiegend unidirektional verstärkten Schaftmaterials und des polykristallinen, keramischen Kopfmaterials gerecht wird.

Zur Lösung dieser Aufgabe wird ein Oberschenkelteil der eingangs genannten Art vorgeschlagen, das dadurch gekennzeichnet ist, daß der Endoprothesenkopf aus polykristallinem, isotropem, keramischem Material besteht, daß das obere Schaftteil im wesentlichen konisch ausgebildet ist und in den Endoprothesenkopf bis in die Kugel hineinragt und daß der zwischen der Kopfachse und der durch die Hauptorientierung der Fasern im unteren Schaftteil gegebenen unteren Schaftachse eingeschlossene Winkel größer ist als der zwischen der oberen Schaftachse und der unteren Schaftachse eingeschlossene Winkel.

Der von der Kopfachse und der oberen Schaftachse eingeschlossene Winkel kann Werte zwischen 0 und 45 Grad, vorzugsweise jedoch zwischen 3 und 35 Grad annehmen.

Eine für sämtliche Konstruktionen vorteilhafte Ausgestaltung der Erfindung besteht darin, daß die Fasern im oberen Schaftteil parallel der konvexen Außenfläche verlaufen und somit von dieser Fläche nicht angeschnitten werden.

Als geeignete Materialien für das Oberschenkelteil werden insbesondere ein kohlenstoffaserverstärkter Kohlenstoff für den Schaft und polykristalliner Kohlenstoff oder ein Siliziumcarbid-Kohlenstoff-Verbundwerkstoff für den Endoprothesenkopf verwendet. Der Verbundwerkstoff besitzt vorzugsweise ein Einlagerungsgefüge aus einer SiC-Matrix und Kohlenstoffkörnern. Die Korngröße der Kohlenstoffkörner liegt im Bereich von 5-200 $\mu$m, vorzugsweise im Bereich von 10-60 $\mu$m.

Die beschriebene Erfindung soll anhand der Zeichnungen nachfolgend näher erläutert werden.

Figur 1 zeigt ein erfindungsgemäßes Oberschenkelteil, bei dem Kopfachse und obere Schaftachse einen spitzen Winkel einschließen.

Figur 2 zeigt einen Schnitt in der Ebene E-F in Fig. 1.

Figur 3 zeigt ein Oberschenkelteil, bei dem Kopfachse und obere Schaftachse entsprechend dem Stand der Technik identisch sind.

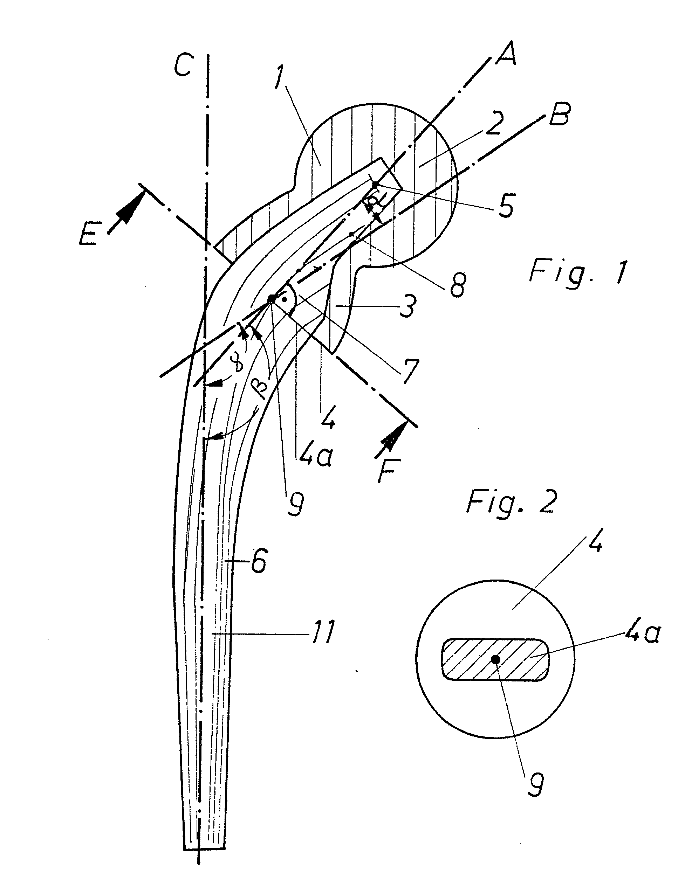

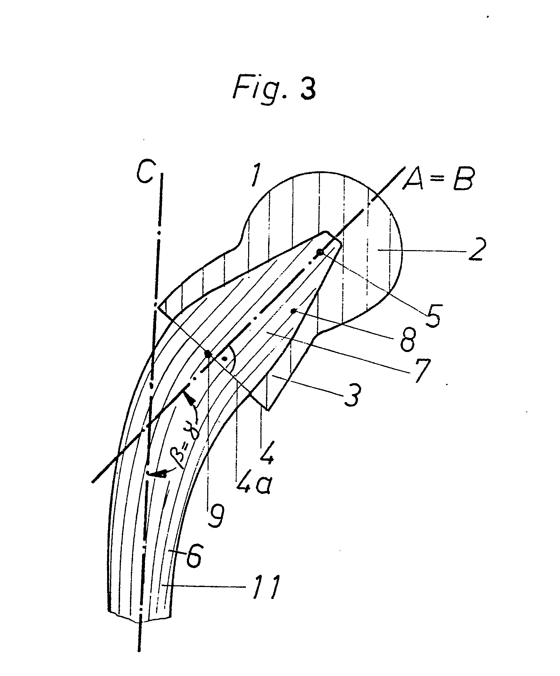

Gemäß Fig. 1 und 3 weist das Oberschenkelteil einen Endoprothesenkopf 1 mit einer Kugel 2 und einem Kragenteil 3 auf. Das Kragenteil dient mit dem Rand der Kopfbasis 4 der Abstützung des Oberschenkelteils auf dem Femurknochen. Die Kopfachse A geht durch den Mittelpunkt 5 der Kugel und steht senkrecht auf der Kopfbasis 4.

Der in den Kopf hineinragende und mit diesem fest verbundene Endoprothesenschaft 6 weist dementsprechend kein Kragenteil auf. Im Endoprothesenschaft kann eine obere Schaftachse B und eine untere Schaftachse C definiert werden. Die obere Schaftachse B ist durch die Richtung der Hauptorientierung der Fasern 8 im oberen Schaftteil 7 und durch den Mittelpunkt 9 der durch die Kopfbasisfläche bestimmten, virtuellen Schnittfläche 4a gegeben. Die untere Schaftachse C ist durch die Hauptorientierung der Fasern im unteren Schaftteil 11 gegeben.

Fig. 1 zeigt die erfindungsgemäße Ausführung, die optimal erscheint. Hierbei schließt die Kopfachse A und die obere Schaftachse B einen spitzen Winkel $\alpha$ ein. Die Fasern verlaufen im oberen Schaftteil parallel zur konvexen Außenfläche des Schaftes. Der zwischen der Kopfachse A und der unteren Schaftachse C eingeschlossene Winkel $\beta$ ist größer als der von der unteren Schaftachse C und der oberen Schaftachse B eingeschlossene Winkel $\gamma$.

Bei dieser Konstruktion sind auf der durch Zug beanspruchten Seite des Schaftes keine Fasern angeschnitten. Die aus der Normalkraft resultierenden Biegekräfte werden in voller Höhe auf den Schaft übertragen, ohne daß der Kopf entscheidend auf Zug bzw. Biegung beansprucht wird. Somit sind sowohl der Schaft als auch der Kopf optimal belastet.

Fig. 3 schließlich zeigt zum Vergleich eine Ausführung nach dem Stand der Technik, die zwar ebenfalls den aus Kugel- und Kragenteil bestehenden Endoprothesenkopf aufweist, bei der jedoch Kopfachse und obere Schaftachse identisch sind, bzw. bei denen die Winkel $\beta$ und $\gamma$ gleich sind.

Auch bei einer derartigen Konstruktion ist wie bei den Ausführungen nach Fig. 1 und 2 als günstig zu beurteilen, daß die auf den Prothesenkopf bei Belastung einwirkende Normalkraft nur relativ geringe Druckkräfte auf den Schaft senkrecht zur Faserorientierung zur Folge hat. Der Schaft wird überwiegend auf Biegung beansprucht, was dem unidirektional verstärkten Material entgegenkommt. Simultan mit dieser Kraftzerlegung sind die Kerbspannungen am oberen Teil der Aussparung des Kopfes erträglich, so daß diese Lösung auch in Bezug auf die Beanspruchung des Prothesenkopfes günstig erscheint. Der nicht zu akzeptierende Nachteil dieser Konstruktion liegt jedoch darin, daß die auf Zug beanspruchten Fasern auf der Außenseite des Schaftes infolge der Verjüngung des Schaftes im Kopf angeschnitten sind, so daß der Prothesenkopf außerordentlich hohen Zugbeanspruchungen unterworfen ist; diese können zum Versagen führen.

Das erfindungsgemäße Oberschenkelteil ist nicht auf bestimmte Schenkelhalshöhen (= Abstand Kopfbasis — Kugelscheitelpunkt) beschränkt. Durch die geeignete Wahl des Winkels zwischen Kopfachse A und oberer Schaftachse B kann jede beliebige Schenkelhalshöhe eingestellt werden, und zwar bei Einhaltung eines festen Winkels. Die gebräuchlichste Schenkelhalshöhe von 50 mm ergibt sich bei einem Winkel α von 17 Grad, eine Schenkelhalshöhe von 56 mm bei α = 14 Grad und 44 mm bei α = 22 Grad.

Obwohl die Hauptorientierungsrichtung der Fasern somit im oberen Schaftteil optimal der Aufnahme der Biegekräfte angepaßt ist, bleibt bei diesen verschiedenen Oberschenkelteilen der äußerlich erkennbare Winkel β, z. B. mit 138 Grad, stets gleich.

**Ansprüche**

1. Oberschenkelteil einer Hüftgelenkendoprothese, bestehend aus einem faserverstärkten, gekrümmten Endoprothesenschaft (6) mit unidirektionaler oder teilweise unidirektionaler Anordnung der Fasern (8) in Längsrichtung des Schaftes, der ein oberes Schaftteil (7), ein stark gekrümmtes Mittelteil und ein sich zum freien Ende hin verjüngendes unteres Schaftteil (11) umfaßt, und einem aus Kugel (2) und Kragenteil (3) bestehenden Endoprothesenkopf (1), wobei der mit dem Endoprothesenkopf (1) fest verbundene Endoprothesenschaft (6) keinen Kragen aufweist und wobei die durch die Senkrechte durch den Kugelmittelpunkt (5) auf die Kopfbasis (4) gegebene Kopfachse (A) und die durch die Hauptorientierung der Fasern (8) im oberen Schaftteil (7) und den Mittelpunkt (9) der durch die Kopfbasisfläche bestimmten virtuellen Schnittfläche (4a) durch den Schaft (6) gegebene obere Schaftachse (B) nicht identisch sind, wobei die Kopfachse (A) und die obere Schaftachse (B) einen spitzen Winkel (α) einschließen, dadurch gekennzeichnet, daß der Endoprothesenkopf (1) aus polykristallinem, isotropem, keramischem Material besteht, daß das obere Schaftteil (7) im wesentlichen konisch ausgebildet ist und in den Endoprothesenkopf (1) bis in die Kugel (2) hineinragt und daß der zwischen der Kopfachse (A) und der durch die Hauptorientierung der Fasern (8) im unteren Schaftteil (11) gegebenen unteren Schaftachse (C) eingeschlossene Winkel (β) größer ist als der zwischen der oberen Schaftachse (B) und der unteren Schaftachse (C) eingeschlossene Winkel (γ).

2. Oberschenkelteil einer Hüftgelenkendoprothese nach Anspruch 1, dadurch gekennzeichnet, daß der von Kopfachse (A) und oberer Schaftachse (B) eingeschlossene Winkel (α) einen Wert zwischen 0 und 45 Grad besitzt.

3. Oberschenkelteil einer Hüftgelenkendoprothese nach Anspruch 1, dadurch gekennzeichnet, daß der von Kopfachse (A) und oberer Schaftachse (B) eingeschlossene Winkel (α) einen Wert zwischen 3 und 35 Grad besitzt.

4. Oberschenkelteil einer Hüftgelenkendoprothese nach einem der Ansprüche 1 bis 3, dadurch gekennzeichnet, daß die Fasern (8) im oberen Schaftteil (7) parallel der konvexen Außenfläche verlaufen.

5. Oberschenkelteil einer Hüftgelenkendoprothese nach einem der Ansprüche 1 bis 3, dadurch gekennzeichnet, daß der Endoprothesenschaft (6) aus einem kohlenstofffaserverstärkten Kohlenstoffmaterial besteht.

6. Oberschenkelteil einer Hüftgelenkendoprothese nach einem der Ansprüche 1 bis 3, dadurch gekennzeichnet, daß der Endoprothesenkopf (1) aus polykristallinem Kohlenstoff besteht.

7. Oberschenkelteil einer Hüftgelenkendoprothese nach einem der Ansprüche 1 bis 3, dadurch gekennzeichnet, daß der Endoprothesenkopf (1) aus einem Siliziumcarbid-Kohlenstoff-Verbundwerkstoff besteht.

**Claims**

1. Upper thigh part of a hip-joint endoprosthesis, consisting of a fibre-strengthened, curved endoprosthesis shaft (6) with unidirectional or partially unidirectional arrangement of the fibres (8) in the longitudinal direction of the shaft, which comprises an upper shaft part (7), a sharply curved central part and a lower shaft part (11) which tapers toward the free end, and of an endoprosthesis head (1) consisting of ball (2) and collar section (3), in which the endoprosthesis shaft (6) which is firmly connected with the endoprosthesis head (1) does not have a collar, and in which the head axis (A) given by the perpendicular line through the central point (5) of the ball onto the base (4) of the head, and the upper shaft axis (B), given by the principal orientation of the fibres (8) in the upper shaft part (7) and the central point (9) of the virtual intersecting plane (4a) through the shaft (6) determined through the basal plane of the head, are not identical, in which the head axis (A) and the upper shaft axis (B) form an acute angle (α), characterized in that the endoprosthesis head (1) consists of polycrystalline, isotropic, ceramic material, that the upper shaft part (7) is constructed substantially conically and protrudes into the endoprosthesis head (1) up into the ball (2), and that the angle (β) formed between the head axis (A) and the lower shaft axis (C), given by the principal orientation of the fibres (8) in the lower shaft part (11), is greater than the angle (γ) formed between the upper shaft axis (B) and the lower shaft axis (C).

2. Upper thigh part of a hip-joint endoprosthesis according to Claim 1, characterized in that the angle (α) formed by head axis (A) and upper shaft axis (B) has a value of between 0 and 45 degrees.

3. Upper thigh part of a hip-joint endoprosthesis according to Claim 1, characterized in that the angle (α) formed by head axis (A) and upper shaft axis (B) has a value of between 3 and 35 degrees.

4. Upper thigh part of a hip-joint endoprosthesis according to one of Claims 1 to 3, characterized in that the fibres (8) in the upper shaft part (7) run parallel to the convex outer surface.

5. Upper thigh part of a hip-joint endoprosthesis according to one of Claims 1 to 3, characterized in that the endoprosthesis shaft (6) consists of a carbon material strengthened with carbon fibres.

6. Upper thigh part of a hip-joint endoprosthesis according to one of Claims 1 to 3, characterized in that the endoprosthesis head (1) consists of polycrystalline carbon.

7. Upper thigh part of a hip-joint endoprosthesis according to one of Claims 1 to 3, characterized in that the endoprosthesis head (1) consists of a silicon carbide-carbon composite material.

## Revendications

1. Elément supérieur d'ossature d'une prothèse de l'articulation de la hanche, comportant une tige terminale coudée (6), en matière armée de fibres (8) disposées suivant une orientation unidirectionnelle ou partiellement unidirectionnelle dans le sens de la longueur de la tige (6) ; celle-ci comportant une partie supérieure (7), une partie médiane fortement coudée, et une partie inférieure (11), qui va en s'amincissant vers son extrémité libre ; et une tête d'extrémité de prothèse (1) constituée par un bossage sphérique (2) bordé par un collet (3) ; la tige terminale de prothèse (6) solidement fixée à la tête (1) ne comportant aucun collet ; et l'axe (A) de la tête (1), passant par le centre (5) du bossage sphérique (2) et perpendiculaire à la base (4) de la tête, n'étant pas confondu avec la direction principale des fibres de renfort (8) de la partie supérieure (7) de la tige terminale (6), à l'endroit du centre (9) de la surface d'intersection virtuelle (4a) déterminée par l'entrée de la tige (6) à la base de la tête (1), suivant l'axe (B) de la partie supérieure (7) de la tige (6) ; l'axe (A) de la tête et l'axe (B) de la partie supérieure (7) de la tige (6) formant l'un par rapport à l'autre un angle aigu (α) ; caractérisé en ce que la tête d'extrémité de prothèse (1) est réalisée en un matériau céramique, polycristallin et isotrope ; en ce que la partie supérieure (7) de la tige (6) est sensiblement conique, et se trouve engagée en position de service dans la tête (1) jusqu'au bossage sphérique (2) et en ce que l'angle (β), formé entre l'axe (A) de la tête (1) et l'axe (C), déterminé dans la partie inférieure (11) de la tige par l'orientation principale des fibres de renfort (8), est supérieur à l'angle (γ), formé entre l'axe (B) de la partie supérieure (7) de la tige (6) et l'axe (C) de la partie inférieure (11) de celle-ci.

2. Elément supérieur de prothèse de la hanche selon la revendication 1, caractérisé en ce que l'angle (α), formé entre l'axe (A) de la tête et l'axe (B) de la partie supérieure (7) de la tige est compris entre 0 et 45 degrés.

3. Elément supérieur de prothèse de la hanche selon la revendication 1, caractérisé en ce que l'angle (α), formé entre l'axe (A) de la tête et l'axe (B) de la partie supérieure (7) de la tige, est compris entre 0 et 35 degrés.

4. Elément supérieur de prothèse de la hanche selon la revendication 1, caractérisé en ce que les fibres de renfort (8) de la partie supérieure (7) de la tige sont parallèles à la surface convexe externe de celle-ci.

5. Elément supérieur de prothèse de la hanche selon l'une des revendications 1 à 3, caractérisé en ce que la tige terminale (6) est réalisée en un matériau à base de carbone, renforcé par des fibres de carbone.

6. Elément supérieur de prothèse de la hanche selon l'une des revendications 1 à 3, caractérisé en ce que la tête d'extrémité de prothèse (1) est réalisée en un matériau à base de carbone polycristallin.

7. Elément supérieur de prothèse de la hanche selon l'une des revendications 1 à 3, caractérisé en ce que la tête d'extrémité de prothèse (1) est réalisée en un matériau composite, à base de carbure de silicium et de carbone.

0 025 835

Fig. 1

Fig. 2

*Fig.* 3